# EUROPEAN PATENT APPLICATION

(11) **EP 3 075 409 A1**
(43) Date of publication of application: **05.10.2016**
(21) Application number: 14866563.1
(22) Date of filing: 11.09.2014
(51) Int. Cl.: A61M 25/10, A61B 18/12, A61M 25/14

(54) **CATHETER**

(30) Priority: 29.11.2013 JP 2013247454
(71) Applicant: Olympus Corporation, Tokyo 192-8507 (JP)
(72) Inventor: KIYOKAWA, Madoka, Tokyo 192-8507 (JP); YANUMA, Yutaka, Tokyo 192-8507 (JP)
(74) Representative: Schicker, Silvia
(86) International application number: PCT/JP2014/074074
(87) International publication number: WO 2015/079774

(57) **Abstract**

A catheter includes: a tubular insertion section configured to inserted into a body; an expandable and contractible balloon that is disposed at a distal end portion of the insertion section to communicate with lumen of the insertion section; a knife wire that is disposed at the distal end portion of the insertion section and configured to treat a biological tissue; and an operation section that is disposed at a proximal end portion of the insertion section and configured to operate the balloon and the knife wire. The operation section includes a connecting portion that is fitted to a fluid discharge port of a pressurizer capable of supplying a fluid for causing the balloon to expand and a fixing tool which is configured to fix the operation section to the pressurizer to be detachable therefrom.

## Description

### [Technical Field]

The present invention relates to a medical catheter.

Priority is claimed on Japanese Patent Application No. 2013-247454, filed November 29, 2013, the contents of which is incorporated herein by reference.

### [Background Art]

In the related art, a type of treatment performed using a balloon catheter in which a balloon catheter body having an expandable and contractible balloon and a pressurizer attached to the balloon catheter to cause the balloon to expand are combined is known (for example, see Patent Literatures 1 and 2). In general, there are many types of balloon catheter bodies depending on differences in balloon diameter, balloon length, burst pressure, and the like, and thus hospitals normally stock various types of balloons in preparation for various cases. On the other hand, since two types of pressurizers for a small balloon (for example, with a volume of about 20 cc) and a large balloon (for example, with a volume of about 60 cc) can cope with most balloons, only the two types of pressurizers need to be stocked.

For example, Patent Literature 1 discloses a pressurizer including a pressurizer body, a plunger, a pressure gauge, and a connection tube.

In treatment using a balloon catheter, in general, an operator performs an operation of adjusting a position of a balloon using an operation unit disposed on a proximal side of a balloon catheter body and an assistant operates a pressurizer. Accordingly, in a general pressurizer, a connection tube extends for spacing the pressurizer from the operation unit of the balloon catheter and a fluid for causing a balloon to expand and contract flows in the connection tube.

As an example of a treatment tool which is used in combination with an endoscope, a multitask catheter for endoscope having a plurality of treatment functions is disclosed in Patent Literature 3. In treatment using an endoscope, in general, an operator of the endoscope handles only an operation of moving the endoscope and the catheter forward and backward and an assistant performs general operations of the catheter. The multitask catheter described in Patent Literature 3 is provided with a connector for attaching the catheter to the endoscope.

When a treatment tool which is used in combination with an endoscope is a multitask catheter not using a pressurizer, the operations for treatment are carried out using a single operation unit. Accordingly, an assistant operating the treatment tool can hold the operation unit with one hand and can alternately perform the operations with the other hand.

### [Citation List]

### [Patent Literature]

[Patent Literature 1] Japanese Unexamined Patent Application, First Publication No. 2013-094676
[Patent Literature 2] Published Japanese Translation of PCT Application No. 2000-515036
[Patent Literature 3] Japanese Unexamined Patent Application, First Publication No. 2008-302226

### [Summary of Invention]

### [Technical Problem]

Here, when a function of a balloon catheter is added as a function of the multitask catheter, it is necessary to operate the operation unit of the multitask catheter and the pressurizer for causing a balloon to expand and contract for one person. However, when an operator holds the operation unit of the multitask catheter and the pressurizer with both hands, both hands are occupied and thus the operator cannot operate the operation unit and the pressurizer.

Accordingly, in the related art, there is a possibility that an assistant will have to perform considerably troublesome operations of forcibly holding both the pressurizer and the operation unit of the multitask catheter with one hand and performing an additional treatment function with the other hand, or first detaching the pressurizer and then performing an additional treatment function of the catheter, or operating the multitask catheter by two assistants.

When the multitask catheter is attached to the pressurizer using the connector of the multitask catheter described in Patent Literature 3, the connector has flexibility by which positions of the operation unit for each functions can be freely moved and thus it is difficult for one person to operate the multitask catheter.

The present invention is made in consideration of the above-mentioned circumstances and an object thereof is to provide a catheter which can enable an operator to perform a plurality of operations by himself or herself.

### [Solution to Problem]

According to a first aspect of the present invention, a catheter is provided including: a tubular insertion section configured to be inserted into a body; an expandable and contractible balloon that is disposed at a distal end portion of the insertion section to communicate with lumen of the insertion section; a treatment section that is disposed at the distal end portion of the insertion section and configured to treat a biological tissue; and an operation section that is disposed at a proximal end portion of the insertion section and configured to operate the balloon and the treatment section, wherein the operation section includes a connecting portion that is attached to a discharge port of a pressurizer capable of supplying a fluid to the balloon from the discharge port, and a fixing tool which is configured to fix the operation section to the pressurizer to be detachable therefrom.

A second aspect of the present invention provides the catheter according to the first aspect, wherein the fixing tool has a C shape, and the operation section includes a display portion in which at least three outer diameters of the balloon and internal pressures of the balloon corresponding to the at least three outer diameters are displayed on a substantial plane intersecting a plane perpendicular to a center line of a substantial cylinder formed by the C shape of the fixing tool at an angle of 45° or less to exhibit a one-to-one relationship between the outer diameter and the internal pressure.

A third aspect of the present invention provides the catheter according to the second aspect, wherein the relationship between the outer diameter of the balloon and the internal pressure of the balloon is nonlinear.

A fourth aspect of the present invention provides the catheter according to the first aspect, wherein the operation section includes a display portion in which at least three outer diameters of the balloon and internal pressures of the balloon corresponding to the at least three outer diameters are displayed to exhibit a one-to-one relationship between the outer diameter and the internal pressure, the pressurizer includes a gauge indicating a pressure of the fluid in correspondence to the pressure of the fluid, and the fixing tool connects the operation section and the pressurizer to have a positional relationship in which display of the outer diameters and the internal pressures of the balloon in the display portion and the pressure displayed in the gauge are capable of simultaneously being visually recognized from a predetermined direction.

A fifth aspect of the present invention provides the catheter according to the fourth aspect, wherein the relationship between the outer diameter of the balloon and the internal pressure of the balloon is nonlinear.

A sixth aspect of the present invention provides the catheter according to any one of the first to fifth aspects, wherein the fixing tool is attachable to and detachable from the pressurizer and is selectively attachable to and detachable from any one of the pressurizer and an endoscope.

A seventh aspect of the present invention provides the catheter according to any one of the first to sixth aspects, wherein the treatment section includes a knife wire that is exposed to the outside of the insertion section at the distal end portion of the insertion section, that extends to the operation section via the lumen of the insertion section, and configured to incise a biological tissue by supplying a high-frequency current to the biological tissue, the first lumen into which the knife wire is inserted is formed in the insertion section, and the operation section includes a slider that is fixed to a proximal end of the knife wire to cause the knife wire to move forward and backward in the axial direction of the knife wire, and a plug that is fixed to the slider, is electrically connected to the knife wire, and is capable of being connected to a high-frequency power supply for supplying the high-frequency current to the knife wire.

An eighth aspect of the present invention provides the catheter according to any one of the first to seventh aspects, wherein the second lumen into which a guide wire is inserted is formed in the insertion section, and the operation section includes a proximal opening which communicates with the second lumen at a proximal end portion of the second lumen and into which the guide wire is inserted.

### [Advantageous Effects of Invention]

According to the above-mentioned aspects, the proximal operation section of the multitask catheter having an expandable balloon can be attached to and detached from the body (a housing having a holding portion) of the pressurizer. Accordingly, since an assistant can hold the proximal operation section of the catheter while holding the pressurizer, the assistant can alternately perform the operation functions with his or her other hand.

### [Brief Description of Drawings]

Fig. 1 is a side view of a catheter according to an embodiment of the present invention.
Fig. 2 is a cross-sectional view of the catheter.
Fig. 3 is a cross-sectional view taken along line A-A in Fig. 2.
Fig. 4 is a side view illustrating a configuration example of a display portion in the catheter.
Fig. 5 is a perspective view illustrating another configuration example of a display portion in the catheter.
Fig. 6 is a diagram illustrating a step in using the catheter.
Fig. 7 is a diagram illustrating a step in using the catheter.
Fig. 8 is a diagram illustrating a step in using the catheter.
Fig. 9 is a diagram illustrating a step in using the catheter.
Fig. 10 is a diagram illustrating a step in using the catheter.
Fig. 11 is a diagram illustrating a step in using the catheter.
Fig. 12 is a diagram illustrating a step in using the catheter.
Fig. 13 is a diagram illustrating a step in using the catheter.
Fig. 14 is a diagram illustrating a step in using the catheter.
Fig. 15 is a diagram illustrating a step in using the catheter.
Fig. 16 is a diagram illustrating a step in using the catheter.
Fig. 17 is a diagram illustrating a step in using the catheter.
Fig. 18 is a diagram illustrating a step in using the catheter.
Fig. 19 is a diagram illustrating a step in using the catheter.
Fig. 20 is a diagram illustrating a step in using the catheter.
Fig. 21 is a diagram illustrating a step in using the catheter.
Fig. 22 is a diagram illustrating a step in using the catheter.
Fig. 23 is a diagram illustrating a step in using the catheter.
Fig. 24 is a diagram illustrating a step in using the catheter.
Fig. 25 is a diagram illustrating a step in using the catheter.
Fig. 26 is a diagram illustrating a step in using the catheter.
Fig. 27 is a diagram illustrating a step in using the catheter.
Fig. 28 is a diagram illustrating a step in using the catheter.
Fig. 29 is a diagram illustrating a step in using the catheter.
Fig. 30 is a diagram illustrating a step in using the catheter.
Fig. 31 is a diagram illustrating a step in using the catheter.
Fig. 32 is a diagram illustrating a step in using the catheter.
Fig. 33 is a diagram illustrating a step in using the catheter.
Fig. 34 is a diagram illustrating a step in using the catheter.
Fig. 35 is a diagram illustrating a step in using the catheter.
Fig. 36 is a diagram illustrating a step in using the catheter.
Fig. 37 is a diagram illustrating a step in using the catheter.
Fig. 38 is a diagram illustrating a step in using the catheter.
Fig. 39 is a diagram illustrating a step in using the catheter.
Fig. 40 is a diagram illustrating a step in using the catheter.
Fig. 41 is a diagram illustrating a step in using the catheter.
Fig. 42 is a diagram illustrating a step in using the catheter.
Fig. 43 is a diagram illustrating another usage of the catheter.

### [Description of Embodiments]

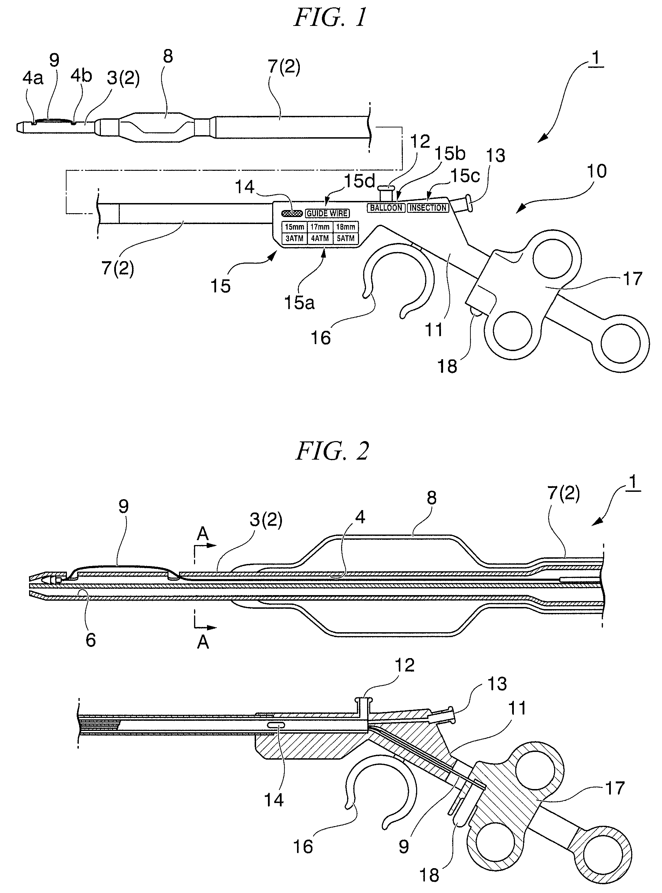

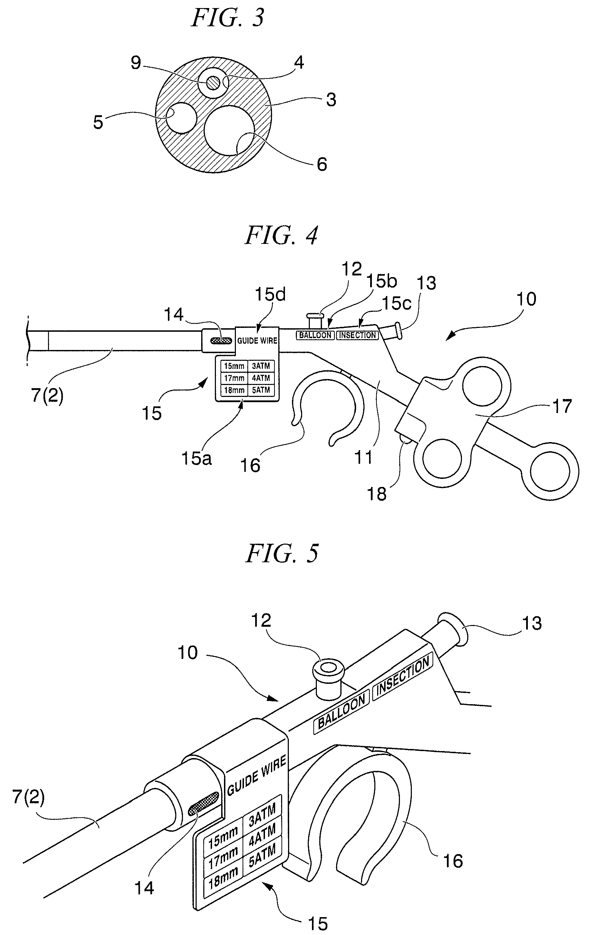

An embodiment of the present invention will be described below. Fig. 1 is a side view of a catheter according to this embodiment. Fig. 2 is a cross-sectional view of the catheter. Fig. 3 is a cross-sectional view taken along line A-A in Fig. 2.

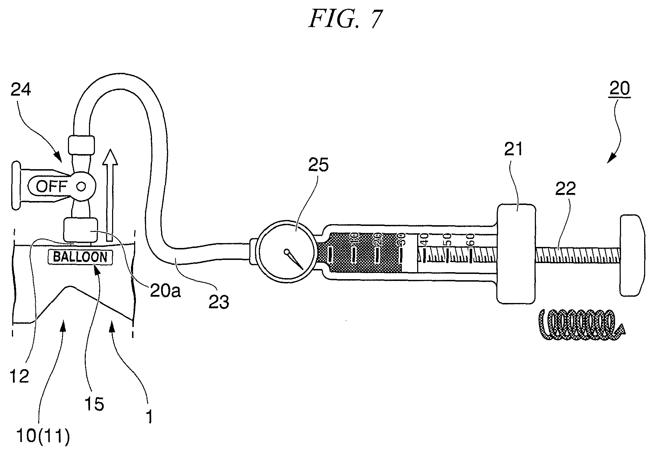

As illustrated in Figs. 1 and 2, the catheter 1 according to this embodiment includes an insertion section 2, an expandable and contractible balloon 8, a knife wire 9 (treatment section), and an operation section 10. The catheter 1 can be connected to a pressurizer 20 (see Fig. 7) for causing the balloon 8 to expand and contract. The configuration of the pressurizer 20 is not particularly limited as long as it can supply a fluid to the balloon 8 to cause the balloon 8 to expand and contract. For example, in this embodiment, the pressurizer 20 illustrated in Fig. 7 includes a cylinder 21, a plunger 22 that is screwed into the cylinder 21, a tubular connection tube 23 that is fixed to the cylinder 21, and a three-way stopcock 24 that is disposed at a distal end of the connection tube 23. Accordingly, a fluid in the cylinder 21 can be extruded using the plunger 22 and can be discharged from a discharge port (in this embodiment, a port of the three-way stopcock 24 not connected to the connection tube 23 is the discharge port) 20a disposed at the distal end of the connection tube 23. In this embodiment, the cylinder 21 is provided with a gauge 25 for displaying a pressure of the fluid in the cylinder 21.

The insertion section 2 is a tubular member that can be inserted into a body. The insertion section 2 includes an inner sheath 3 and an outer sheath 7.

As illustrated in Fig. 3, the inner sheath 3 is constituted as a multi-lumen tube having a first lumen 4, a second lumen 5, and a third lumen 6 formed therein.

The multi-lumen tube constituting the inner sheath 3 is formed of a flexible material having thermal resistance. In this embodiment, the inner sheath 3 is formed of polytetrafluoroethylene (PTFE).

The first lumen 4 of the inner sheath 3 is a passage for a knife wire 9. The first lumen 4 is opened laterally at two positions (a first side hole 4a and a second side hole 4b) in the vicinity of a distal end of the inner sheath 3. The knife wire 9 passes through the two openings on the distal-end side of the first lumen 4.

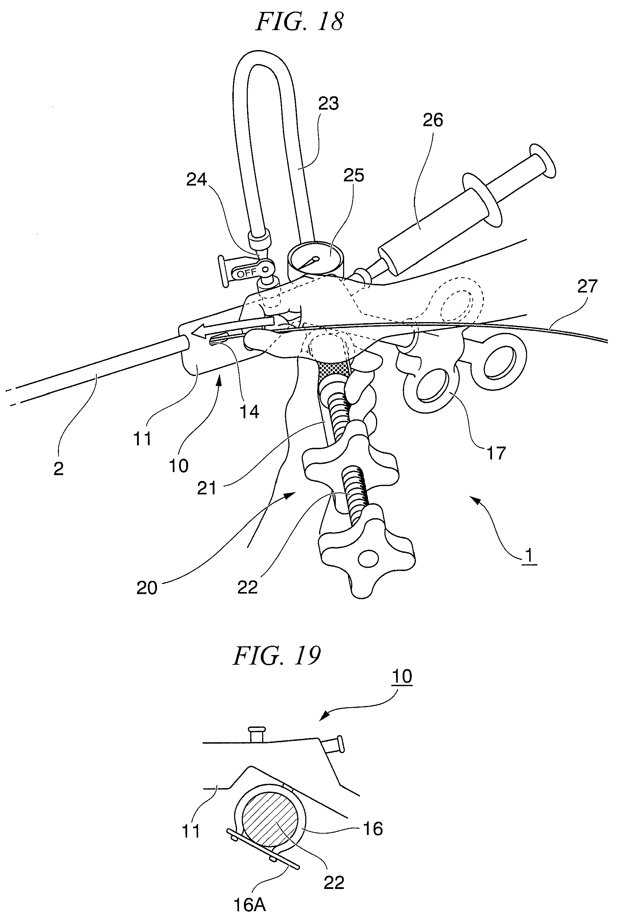

A guide wire 27 (see Fig. 18) can be inserted into the second lumen 5 of the inner sheath 3. A proximal end of the second lumen 5 communicates with a proximal opening 14 in the operation section 10.

The third lumen 6 of the inner sheath 3 serves as a flow channel of a liquid such as a radiocontrast agent. The distal end of the third lumen 6 is opened at the distal end face of the inner sheath 3 and the proximal end of the third lumen 6 is connected to a connector 13 as a liquid feed port through a pipe.

The outer sheath 7 is a tubular member into which the inner sheath 3 is inserted.

The distal end of the outer sheath 7 is water-tightly fixed to the proximal end of the balloon 8. The proximal end of the outer sheath 7 is water-tightly fixed to the distal end of a connecting portion 12 of the operation section 10.

As illustrated in Fig. 2, the balloon 8 is disposed at the distal end of the insertion section 2.

The distal end of the balloon 8 is water-tightly fixed to the outer circumferential surface on the proximal side relative to the second side hole 4b of the inner sheath 3. The proximal end of the balloon 8 is water-tightly fixed to the outer circumferential surface of the distal end of the outer sheath 7. Accordingly, the gap between the inner sheath 3 and the outer sheath 7 is a lumen of the insertion section 2 and serves as a flow channel of a fluid for causing the balloon 8 to expand.

In this embodiment, a correlation between the outer diameter of the balloon 8 and the internal pressure of the balloon 8 is nonlinear. The balloon 8 is a compliance balloon in which an expansion diameter varies depending on a pressure. Most preferably, the material of the balloon 8 is polyamide, polyamide elastomer, PET elastomer, or polyurethane.

In the balloon 8 according to this embodiment formed of these materials, compliance is set to, for example, 15 mm/3 ATM, 17 mm/4 ATM, or 18 mm/5 ATM.

Although the expansion force of the balloon 8 is very small, the balloon may be formed of a material such as latex having very high flexibility.

The knife wire 9 is a conductive wire member which can incise a biological tissue by supplying a high-frequency current to the biological tissue. The knife wire 9 is exposed to the outside of the insertion section 2 at the distal end of the insertion section 2 and extends to the operation section 10 through the lumen of the insertion section 2. In the knife wire 9, a portion that is disposed at the distal end of the insertion section 2 and is exposed to the outside of the insertion section 2 contributes to incision of a biological tissue. In this embodiment, the knife wire 9 can be used for treatment of incising a duodenal papilla PV.

The distal end of the knife wire 9 is locked into the first lumen 4. The distal portion of the knife wire 9 leaves the inner sheath 3 from the first lumen 4 via the first side hole 4a formed on the distal side of the inner sheath 3, enters the first lumen 4 again from the second side hole 4b formed on the proximal side of the inner sheath 3, and extends to the proximal end of the inner sheath 3 through the first lumen 4. The proximal end of the knife wire 9 is fixed to a slider 17 in the operation section 10.

As illustrated in Figs. 1 and 2, the operation section 10 is disposed at the proximal end of the insertion section 2. The operation section 10 is disposed to operate the balloon 8 and the knife wire 9.

The operation section 10 includes a body 11, a connecting portion 12, a display portion 15, a fixing tool 16, a slider 17, a plug 18, and a proximal opening 14.

The connecting portion 12, the connector 13, and the proximal opening 14 are formed in the body 11.

As illustrated in Fig. 7, the discharge port 20a of the pressurizer 20 can be water-tightly attached to the connecting portion 12. By attaching the discharge port 20a of the pressurizer 20 to the connecting portion 12, a fluid for causing the balloon 8 to expand can be introduced from the pressurizer 20 into the balloon 8.

The connector 13 disposed in the body 11 is, for example, a Luer-lock connector and is fixed to the proximal end of the third lumen 6 to communicate with the third lumen 6. For example, a syringe 26 supplying a radiocontrast agent can be attached to the connector 13.

The proximal opening 14 is an opening that communicates with the second lumen 5 at the proximal end of the second lumen 5 and into which a guide wire 27 can be inserted. The guide wire 27 is used as a guide for guiding the distal-end portion of the insertion section 2 of the catheter 1 to a treatment target part T. A known guide wire 27 may be appropriately selected and applied to the catheter 1 according to this embodiment.

As illustrated in Fig. 1, a display portion 15a is a label in which at least three outer diameters of the balloon 8 and internal pressures of the balloon 8 corresponding to the at least three outer diameters are displayed to indicate a one-to-one relationship (hereinafter referred to as compliance) between the outer diameter and the internal pressure, and is attached to the body 11.

In this embodiment, a display portion 15b is an indicator indicating a balloon port to be connected to the pressurizer 20 and is attached to the vicinity of the connecting portion 12. A display portion 15c is an indicator indicating a liquid feed port to be connected to a liquid feed pump or the like and is attached to the vicinity of the connector 13. A display portion 15d is an indicator indicating an insertion port of the guide wire 27 and is attached to the vicinity of the proximal opening 14.

As illustrated in Figs. 4 and 5, the display portions 15a and 15d, which are two of the display portions 15a to 15d, may be displayed as a tag. In this tag, the compliance table is displayed in the planar portion of a plate member fixed to the body 11. In the gu, as illustrated in Fig. 4, the display portions 15a and 15d may be displayed or other information may be displayed. When some of the display portions 15a to 15d are embodied as a tag, there is an advantage in that the size and weight of the operation section 10 can be decreased and an easily visible display can be embodied.

The fixing tool 16 is a portion of the body 11 which is formed in a C shape with which the operation section 10 can be detachably fixed to the pressurizer 20. The body 11 and the fixing tool 16 are set in a relative position to have rigidity at which they are not substantially deformed when a force for operating the slider 17 or the pressurizer 20 is applied to the slider 17 or the pressurizer 20. For example, the body 11 and the fixing tool 16 are formed by integral plastic molding or a combination of plastic-molded components.

The fixing tool 16 can be attached to and detached from the pressurizer 20 and can be selectively attached to and detached from one of the pressurizer 20 and an endoscope 30. That is, when the catheter 1 and the pressurizer 20 are not simultaneously used but the catheter 1 and the endoscope 30 are simultaneously used, the fixing tool 16 can connect the endoscope 30 and the catheter 1 in a fixed state. Accordingly, it is possible to enable an operator to perform easy operations.

The fixing tool 16 connects the operation section 10 and the pressurizer 20 to have a positional relationship in which display of the outer diameters and the internal pressures of the balloon 8 in the display portions 15a to 15d and the pressure displayed in the gauge 25 of the pressurizer 20 are capable of simultaneously being visually recognized from a predetermined direction. In this embodiment the fixing tool 16 is connected to the cylinder 21 of the pressurizer 20.

Regarding the positional relationship between the fixing tool 16 and the display portions 15a to 15d, the display portions 15a to 15d have a positional relationship in which the compliance is displayed on a substantial plane intersecting the plane perpendicular to the center line of a substantial cylinder formed by the C shape of the fixing tool 16 at an angle of 45° or less.

The slider 17 is connected to the body 11 to be movable forward and backward relative to the body 11 to move the knife wire 9 forward and backward in the axial direction of the knife wire 9. The slider 17 is fixed to the proximal end of the knife wire 9. By causing the slider 17 to slide relative to the body 11, the knife wire 9 exposed to the outside of the inner sheath 3 at the distal-end portion of the insertion section 2 can be tightened in an arch shape or loosened.

The plug 18 is a conductor fixed to the slider 17. The plug 18 is fixed to the proximal end of the knife wire 9. The plug 18 and the knife wire 9 are electrically connected to each other. The plug 18 can be connected to a high-frequency power supply 28 for supplying a high-frequency current to the knife wire 9.

The direction of the plug 18 is set such that a direction in which a connection cord 29 for connecting the high-frequency power supply 28 to the plug 18 is pushed into the plug 18 is perpendicular to the sliding direction of the slider 17.

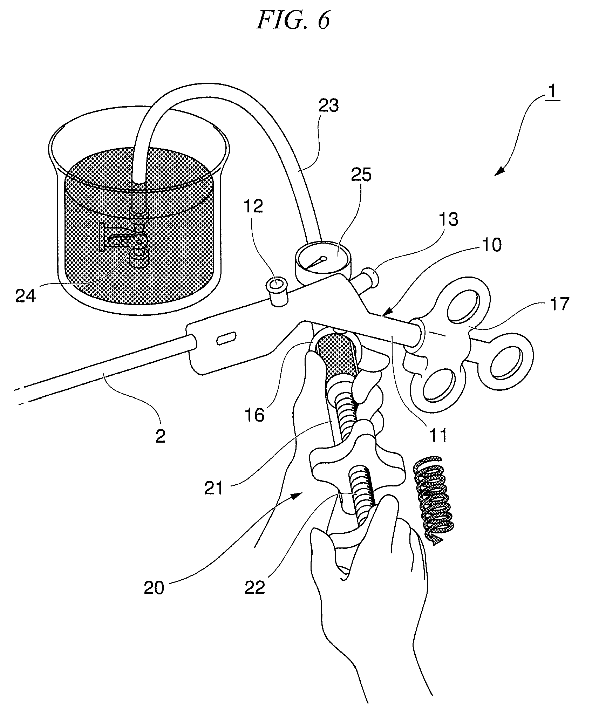

Operations and usage of the catheter 1 according to this embodiment will be described below. Figs. 6 to 42 are diagrams illustrating steps in using the catheter according to this embodiment.

In this embodiment, a calculus can be removed by performing a series of treatments including retrograde cholangiography, incision of duodenal papilla PV, and expansion of duodenal papilla PV using the catheter 1.

First, as illustrated in Figs. 6 and 7, the catheter 1 is connected to the pressurizer 20. Specifically, first, the distal end of the connection tube 23 of the pressurizer 20 is put into a vessel containing an expansion liquid (a fluid including a mixture of a radiocontrast agent and a saline solution), and the plunger 22 of the pressurizer 20 is moved backward to suction the expansion liquid to half the capacity of the pressurizer 20, for example, to 30 cc when the pressurizer 20 can contain 60 cc of the expansion liquid.

The connection tube 23 of the pressurizer 20 is attached to the connecting portion 12 of the operation section 10 via the three-way stopcock 24.

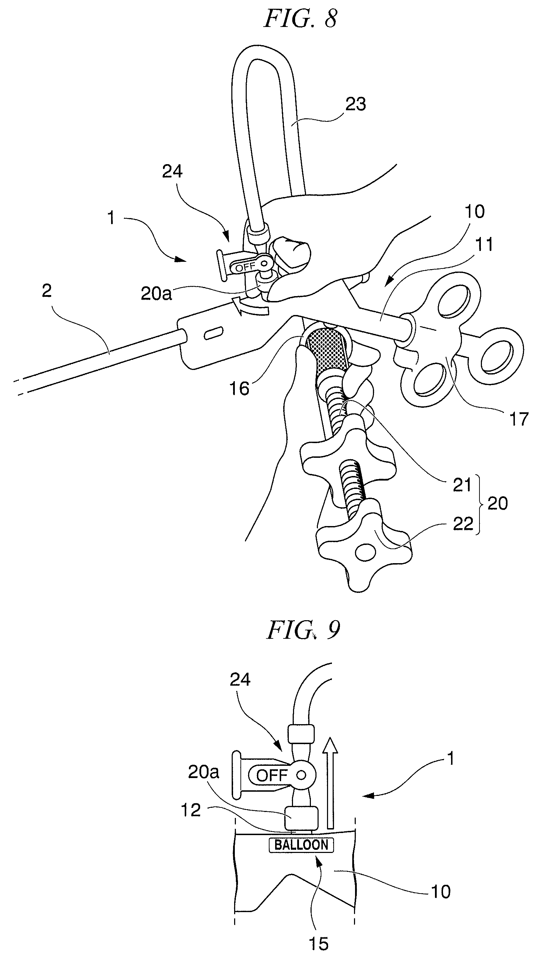

Then, air in the balloon 8 is bled. First, as illustrated in Figs. 8 and 9, the cock of the three-way stopcock 24 is set to a direction in which the catheter 1 and the pressurizer 20 communicate with each other and an open end is closed.

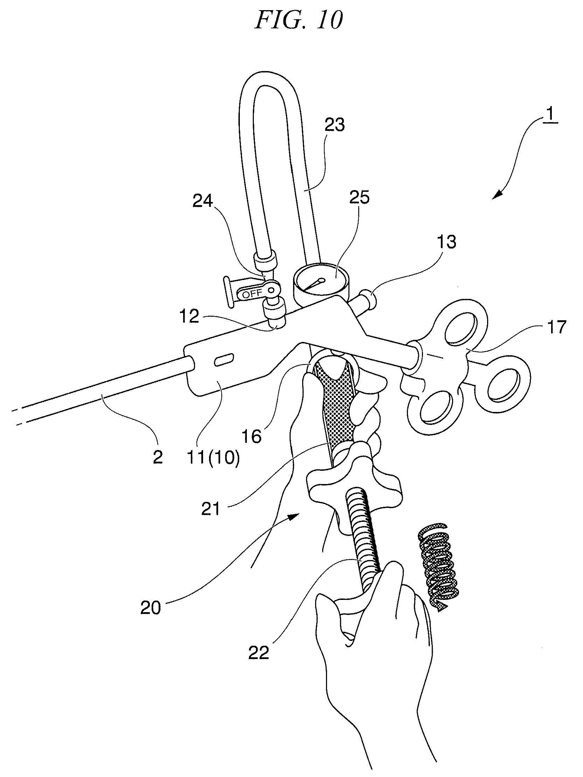

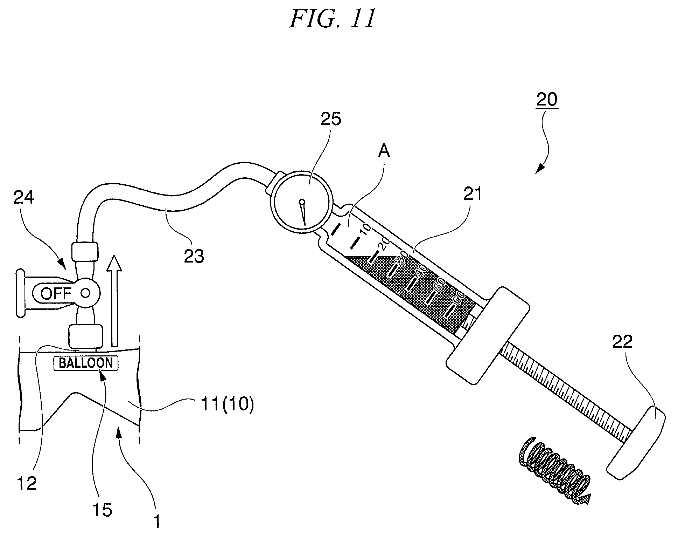

In this state, as illustrated in Fig. 10, the plunger 22 of the pressurizer 20 is moved backward to the full state (for example, the position of 60 cc when he pressurizer 20 can contain 60 cc of the expansion liquid). By this operation, air A between the balloon 8 and the connecting portion 12 (see Fig. 2) is bled into the pressurizer 20 as illustrated in Fig. 11.

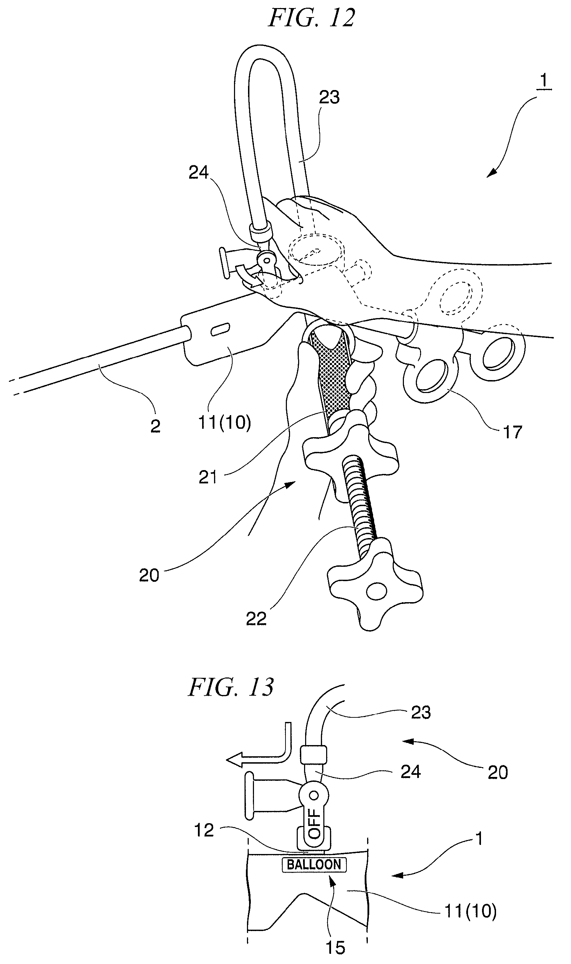

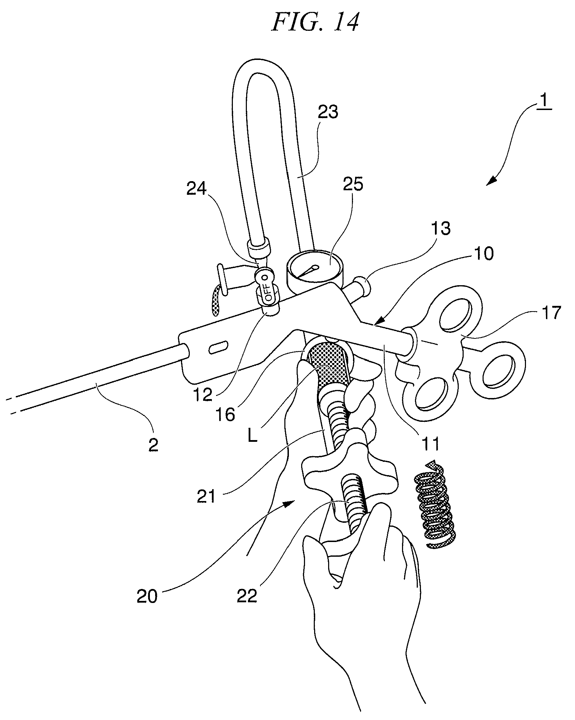

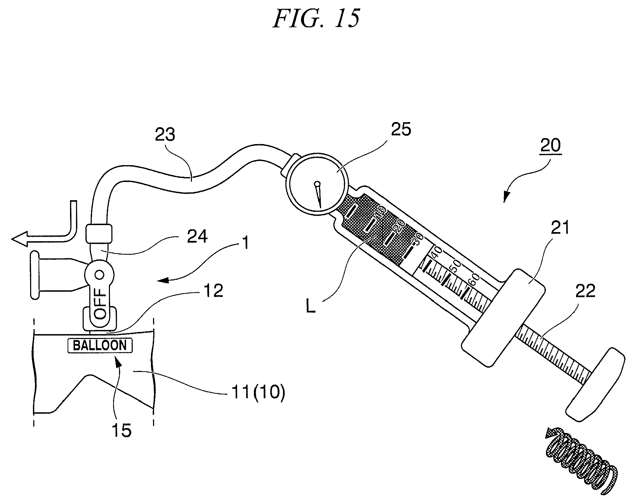

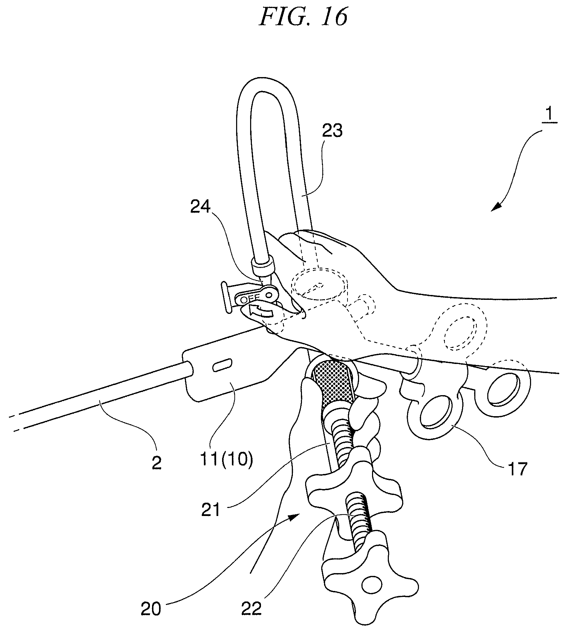

As illustrated in Figs. 12 and Fig. 13, in the state in which the plunger 22 of the pressurizer 20 is moved backward to the position of 60 cc, the cock of the three-way stopcock 24 is rotated in the direction in which the connecting portion 12 is closed and the pressurizer 20 communicates with the open end. Thereafter, as illustrated in Figs. 14 and 15, in a state in which the plunger 22 and the cylinder 21 are inclined such that air (not illustrated) in the pressurizer 20 moves upward, the plunger 22 is moved forward to discharge the air bled into the pressurizer 20 to the outside from the open end of the three-way stopcock 24. At this time, the plunger 22 is moved forward to discharge all of the air in the pressurizer 20 to the outside of the pressurizer 20 until the expansion liquid L is slightly discharged. When the air in the pressurizer 20 is discharged, the cock of the three-way stopcock 24 is rotated in the direction in which the open end is closed and the catheter 1 communicates with the pressurizer 20 as illustrated in Fig. 16.

In order to discharge most of the air in the catheter 1, the bleeding of air is preferably repeated several times (about three times).

When a two-way stopcock is used, the same air bleeding can be performed by removing the connection tube 23 of the pressurizer 20 from the two-way stopcock to discharge the air in the pressurizer 20 in a state in which the end of the two-way stopcock on the catheter 1 side is closed.

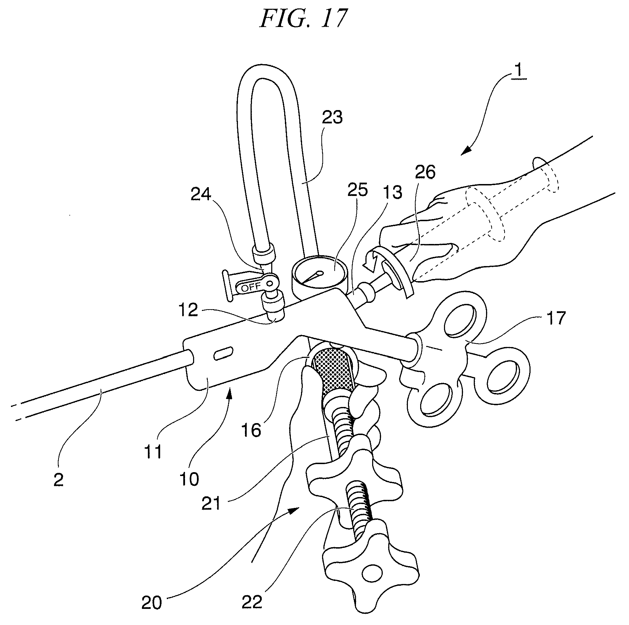

Then, as illustrated in Fig. 17, the syringe 26 for injecting a radiocontrast agent is attached to the connector 13. The syringe 26 for injecting a radiocontrast agent is filled with the radiocontrast agent. The syringe 26 is water-tightly connected to the connector 13, and the radiocontrast agent can be discharged from the distal end of the insertion section 2 by extruding the radiocontrast agent from the syringe 26.

Then, the guide wire 27 for guiding the distal end of the catheter 1 to a treatment target part is prepared. As illustrated in Fig. 18, the guide wire 27 is inserted into the second lumen 5 (see Figs. 2 and 3) from the proximal opening 14 formed in the body 11 of the operation section 10 and is transferred to the opening on the distal-end side of the second lumen 5.

The fixing tool 16 of the catheter 1 is slightly deformed by the elasticity of the fixing tool 16 and receives the cylinder 21 of the pressurizer 20 in the C-shaped portion. When the cylinder 21 is located inside the fixing tool 16, the fixing tool 16 is slightly enlarged by the cylinder 21 and the fixing tool 16 holds the outer circumferential surface of the cylinder 21 by a force with which the fixing tool 16 is restored to the original shape. Accordingly, the fixing tool 16 connects the catheter 1 and the pressurizer 20 with such a holding force that the catheter 1 and the pressurizer 20 do not come out of alignment due to the operation of the syringe 26 or the guide wire 27 in the catheter 1 or the operation of the three-way stopcock 24 of the pressurizer 20.

In order to further enhance the fixing force of the fixing tool 16, both ends of the fixing tool 16 may be connected with an expandable band 16A as illustrated in Fig. 19.

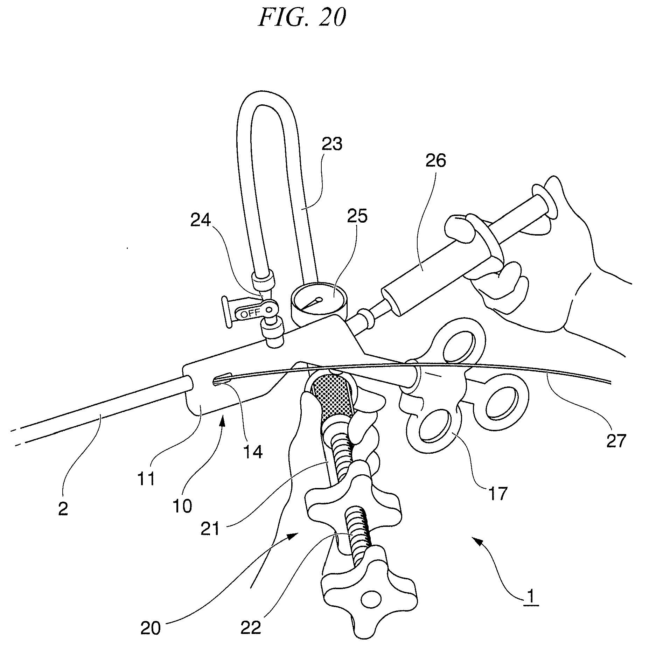

For example, as illustrated in Fig. 20, the operator of the catheter 1 can hold the cylinder 21 of the pressurizer 20 with his or her left hand and can operate the other operation unit (such as the syringe 26, the guide wire 27, the slider 17, and the three-way stopcock 24) with his or her right hand.

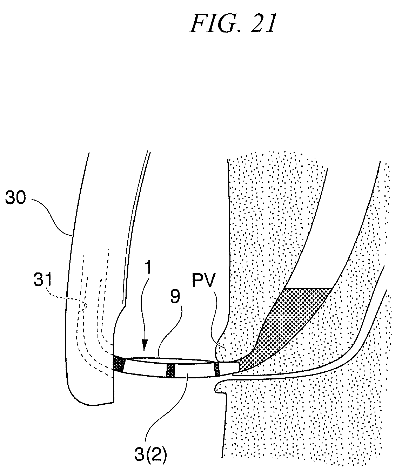

Then, the insertion section 2 of the catheter 1 is inserted into a treatment tool channel 31 of the endoscope 30 from the distal side (see Fig. 21). In this embodiment, the endoscope 30 may be guided to the vicinity of the treatment target portion already before the insertion section 2 of the catheter 1 is inserted into the treatment tool channel 31.

When the distal end of the insertion section 2 of the catheter 1 protrudes from the distal end of the treatment tool channel 31 of the endoscope 30, the distal end of the catheter 1 is inserted into the duodenal papilla PV by operating the endoscope 30 and the catheter 1.

Since the shape of the entrance of the duodenal papilla PV is complex, several techniques are necessary for inserting the distal end of the catheter 1 into the duodenal papilla PV.

As one technique for inserting the distal end of the catheter 1 into the duodenal papilla PV, the direction of the catheter 1 needs to match the direction of a tube in the duodenal papilla PV, that is, the direction of a bile duct. However, the direction of a bile duct cannot be observed by the endoscope 30, and cannot be observed using X-rays in a state in which a radiocontrast agent is not injected. Accordingly, in order to check the direction of the bile duct first, a method of inserting only several mm of the distal end of the catheter 1 into the entrance of the duodenal papilla PV and injecting a slight amount of radiocontrast agent from the syringe 26 attached to the operation section 10 in this state to contrast the bile duct is known (see Figs. 20 and 21). Accordingly, when the direction of the bile duct can be checked, the insertion is attempted with the direction of the insertion section 2 of the catheter 1 matching the direction.

However, since the direction of the bile duct is generally directed in a steeper direction than the direction of the catheter 1 protruding from the endoscope 30, skill is required to operate the endoscope 30 in order to match the direction of the insertion section 2 of the catheter 1 with the direction of the bile duct through only the operation of the endoscope 30.

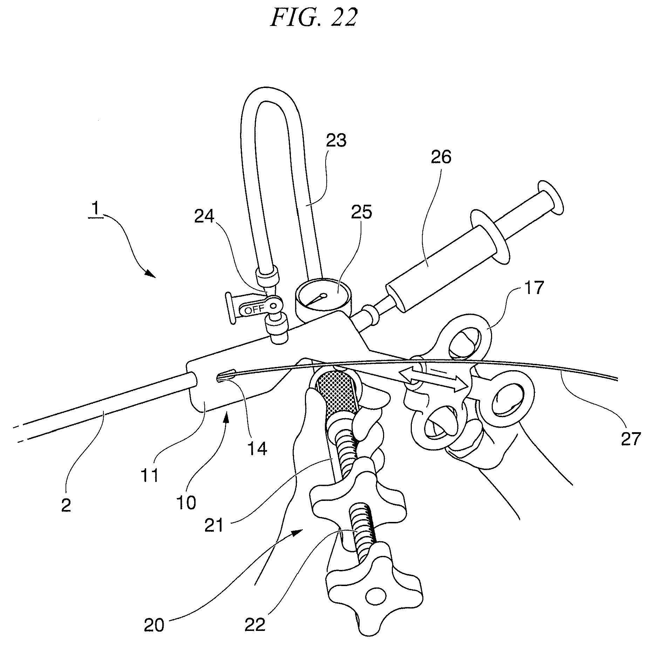

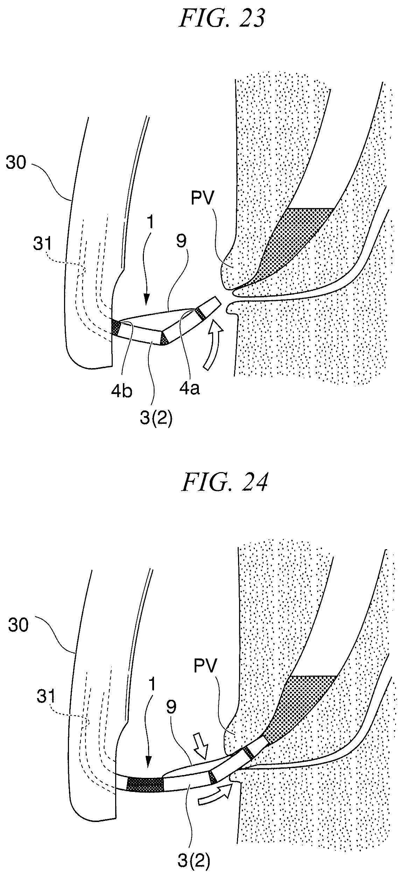

In this embodiment, as illustrated in Figs. 22 and 23, the distal end of the catheter 1 can be bent in an arch shape by operating the operation section 10 of the catheter 1 to pull the knife wire 9 in an arch shape. After the distal end of the catheter 1 goes into the duodenal papilla PV by pulling the knife wire 9 in an arch shape, the knife wire 9 is loosened by reducing the force applied to the slider 17, and the insertion section 2 is further inserted into the bile duct (see Fig. 24). In this way, in this embodiment, by minutely adjusting the direction of the distal end of the insertion section 2 of the catheter 1 by operating the operation section 10, it is possible to more easily match the direction of the insertion section 2 with the direction of the bile duct.

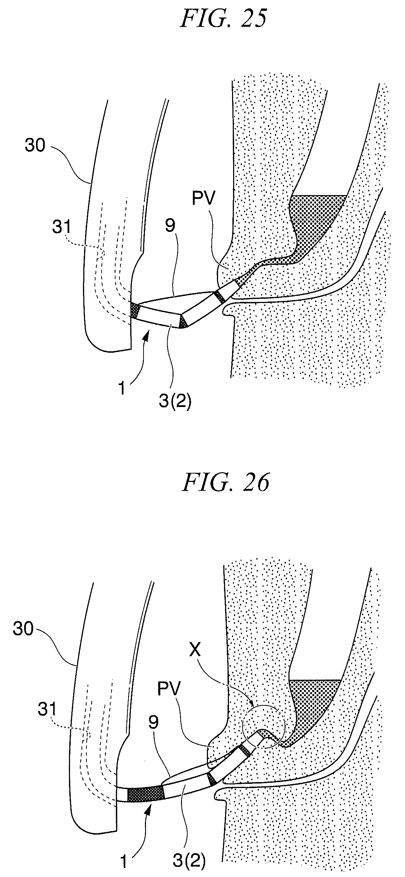

As another technique for inserting the distal end of the catheter 1 into the duodenal papilla PV, the guide wire 27 may be used. A duct in the duodenal papilla PV may be unusually bent as illustrated in Fig. 25. In such a duodenal papilla PV, by only matching the direction of the insertion section 2 of the catheter 1 with the direction of the bile duct, the distal end of the insertion section 2 of the catheter 1 may collide with the bent portion X (see Fig. 26) of the bent lumen from the duodenal papilla PV to the bile duct and thus it may be difficult to insert the insertion section 2 into the bile duct.

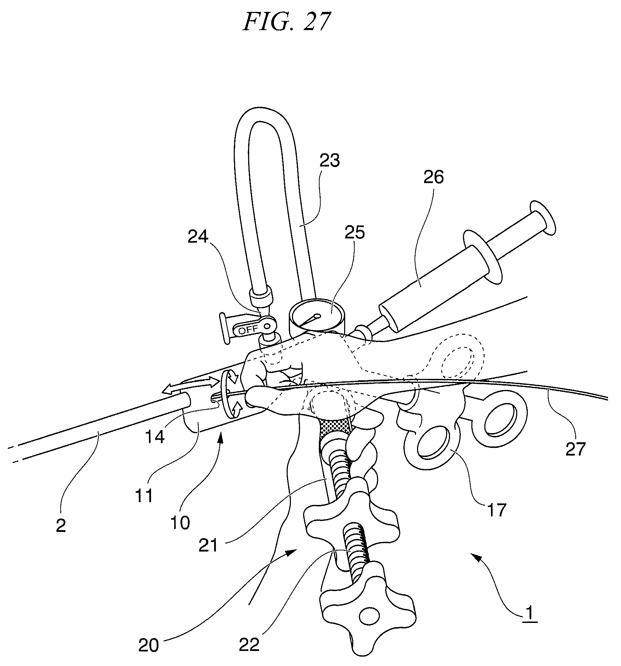

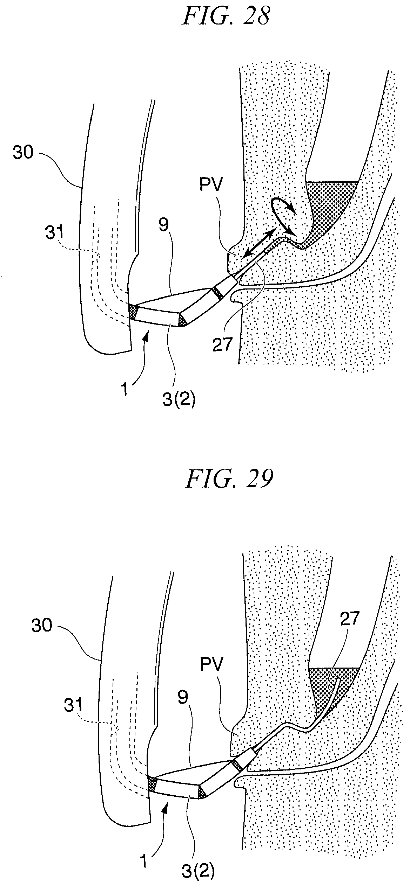

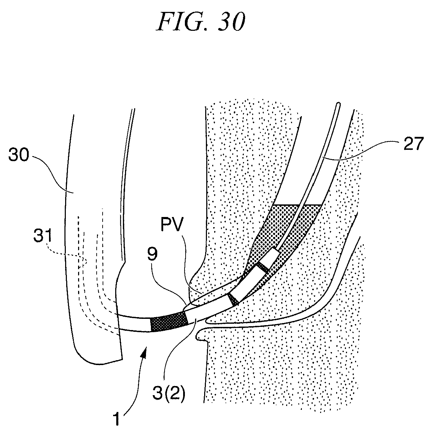

When the distal end of the insertion section 2 of the catheter 1 collides with the bent portion of the bent lumen from the duodenal papilla PV to the bile duct, as illustrated in Figs. 27 and Fig. 28, first, the guide wire 27 is inserted into the bile duct from the duodenal papilla PV using the guide wire 27 having a distal-end portion that is more flexible than the insertion section 2. As a specific operation, the guide wire 27 is caused to slightly protrude from the distal end of the catheter 1 in a state in which the distal end of the insertion section 2 of the catheter 1 is located at the entrance of the duodenal papilla PV (see Fig. 28). By smoothly and slowly moving forward or backward or rotating the proximal-end portion of the guide wire 27 in this state, the direction of the flexible distal end of the guide wire 27 is matched with the bending in the duodenal papilla PV. As a result, the guide wire 27 can pass over the bend in the duodenal papilla PV and advance to the bile duct (see Fig. 29). Accordingly, the guide wire 27 for guiding the catheter 1 is located in the duodenal papilla PV before the catheter 1 is inserted into the duodenal papilla PV. Thereafter, the insertion section 2 of the catheter 1 can be inserted into the deep side of the duodenal papilla PV to follow the guide wire 27 (see Fig. 30).

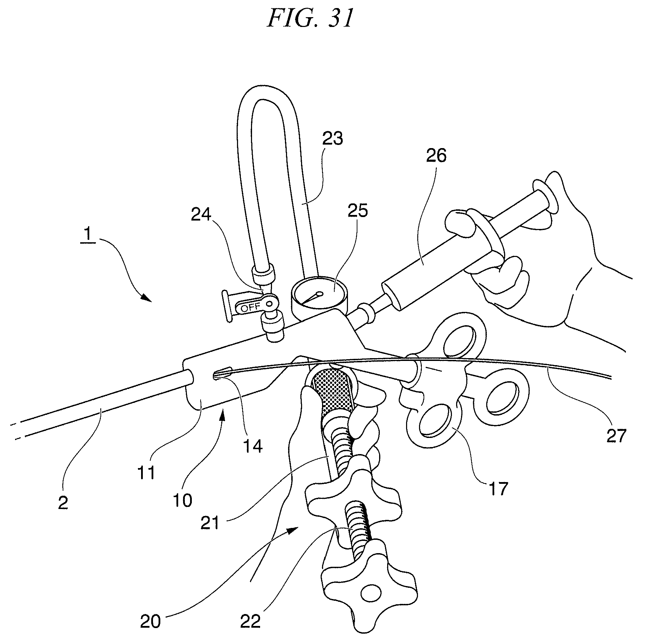

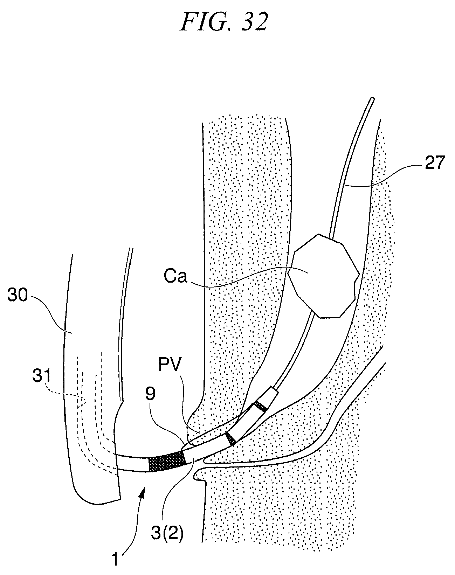

When the insertion section 2 of the catheter 1 is inserted into the bile duct, as illustrated in Fig. 31, the bile duct is filled with the radiocontrast agent from the syringe 26 attached to the operation section 10. When the bile duct is filled with the radiocontrast not transmitting X-rays, only a part of a calculus Ca transmitting X-rays is visualized black by X-rays, and thus it is possible to check the sizes, the number, and the positions of the calculus Ca (see Fig. 32).

In general, the inner diameter of a liquid feed lumen is set to be small to decrease the outer diameter of the catheter 1, and thus a large force has to be continuously applied to the syringe 26 for a predetermined time (several tens of seconds) in order to send an amount of radiocontrast agent to fill the entire bile duct. In this embodiment, the pressurizer 20 is held with the left hand and a force is applied to the syringe 26 to discharge the radiocontrast agent with the right hand.

At this time, since the pressurizer 20 held with the left hand and the syringe 26 to which a force is applied with the right hand are satisfactorily fixed to each other by the fixing tool 16, the syringe 26 is not shaken. Accordingly, a large force can be easily applied to the syringe 26 with the entire right hand, thereby facilitating liquid feed.

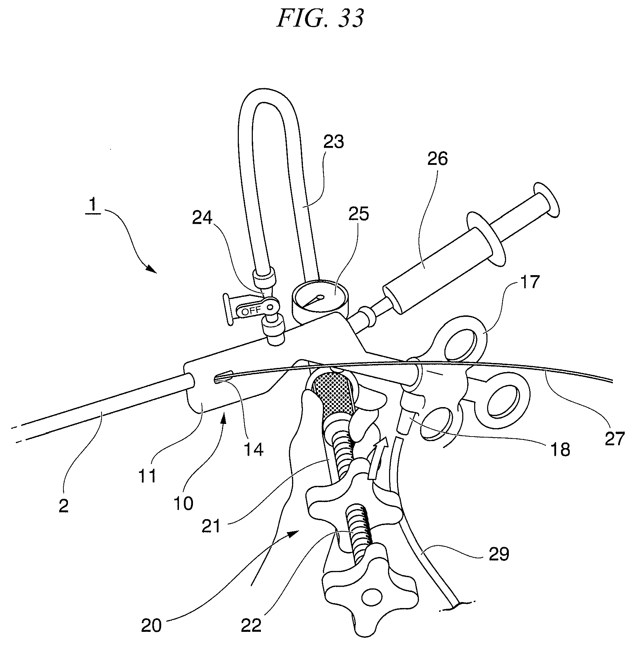

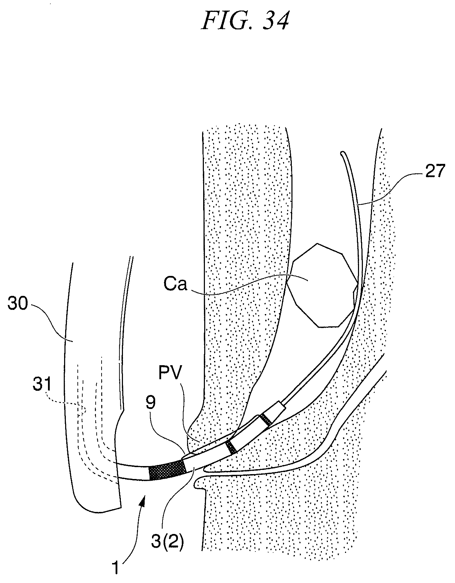

When the bile duct is contrasted and the position of the calculus Ca is checked as illustrated in Fig. 32, the distal-end portion of the knife wire 9 is moved to the incision position of the duodenal papilla PV as illustrated in Fig. 34. As illustrated in Fig. 33, the connection cord 29 of the high-frequency power supply 28 is connected to the plug 18 of the operation section 10.

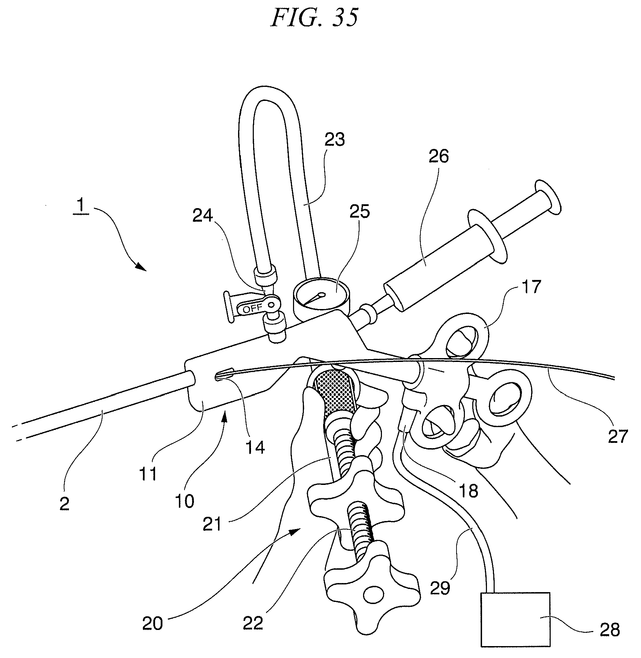

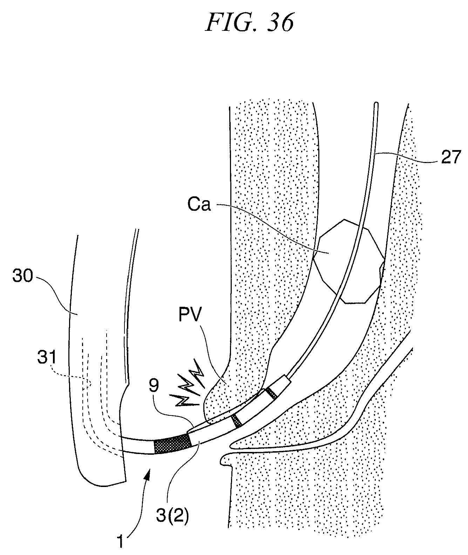

When the high-frequency power supply 28 is connected to the plug 18, the distal-end portion of the knife wire 9 is pulled in an arch shape to come in contact with the duodenal papilla PV as illustrated in Fig. 36 by operating the slider 17 of the operation section 10 illustrated in Fig. 35 and incises the duodenal papilla PV by causing a high-frequency current to flow.

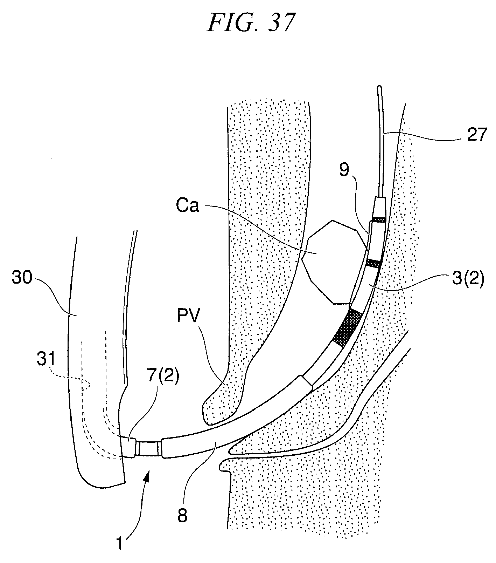

After the duodenal papilla PV is incised using the knife wire 9, the insertion section 2 of the catheter 1 moves forward until substantially the center of the balloon 8 is located at the entrance of the duodenal papilla PV as illustrated in Fig. 37.

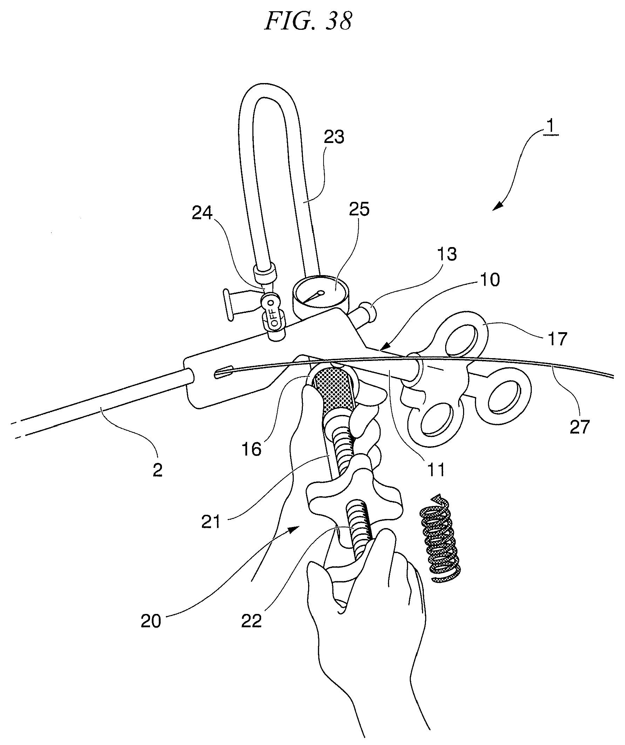

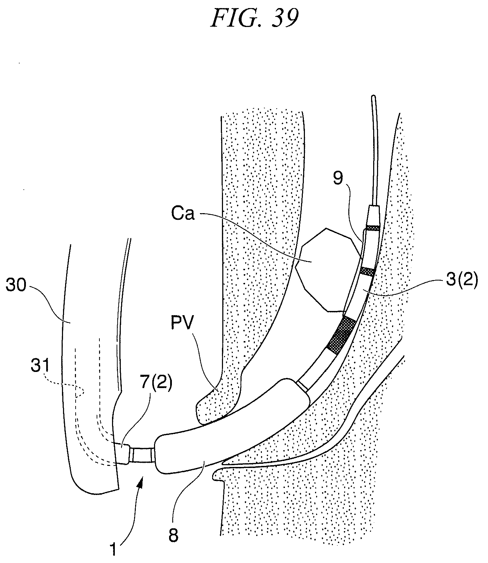

Subsequently, as illustrated in Figs. 38 and 39, the balloon 8 is caused to expand in a state in which the balloon 8 is located in the duodenal papilla PV.

The expansion of the balloon 8 is performed based on the size of a calculus estimated based on an X-ray image and display details of the display portion 15a displayed in the operation section 10 of the catheter 1. In the display portion 15a, the compliance of the balloon 8 (the relationship between the diameter of the balloon 8 and the pressure) is displayed as a table. Accordingly, an operator causing the balloon 8 to expand checks the pressure required for a desired outer diameter using the display of the display portion 15, and determines the value of the pressure with which the diameter of the balloon 8 is substantially equal to the size of the calculus with reference to the compliance table in the display portion 15. Subsequently, the plunger 22 is moved forward to cause the balloon 8 to expand with the pressure determined based on the display of the display portion 15 by the operator as a first target. In this embodiment, in the process of expanding the balloon 8, the compliance table of the display portion 15a and the pressure display of the gauge 25 of the pressurizer 20 are simultaneously visible to the operator. Accordingly, the operator of the pressurizer 20 can easily discern the target pressure and the actual pressure.

When the pressure of the pressurizer 20 approaches a predetermined pressure, the expansion speed is decreased to cause the balloon 8 to expand up to an appropriate size (which may be slightly lower or slightly higher than the predetermined pressure) while the diameter of the balloon 8 and the size of the calculus are directly compared in an X-ray image. In this embodiment, a screwing type is employed in which the fluid for causing the balloon 8 to expand is fed to the balloon 8 by rotating the plunger 22 with respect to the cylinder 21, but the configuration of the plunger 22 and the cylinder 21 are not limited to the screwing type.

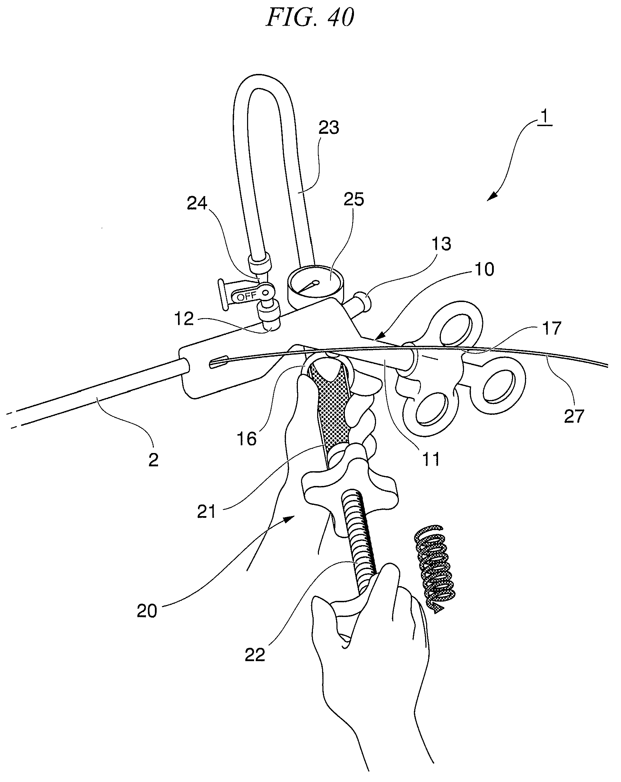

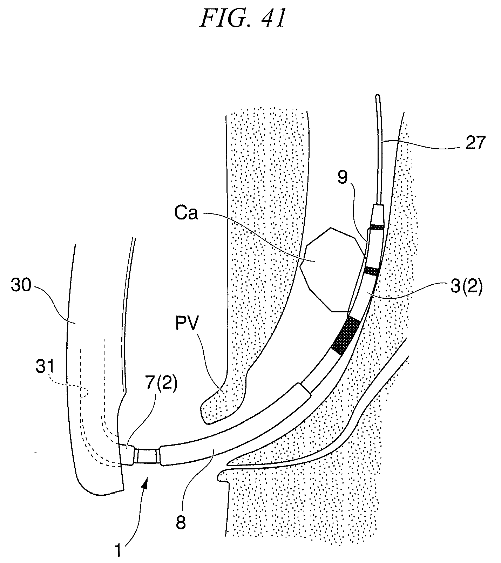

When the duodenal papilla PV is caused to expand up to an appropriate size based on the X-ray image using the balloon 8, the plunger 22 of the pressurizer 20 is moved backward as illustrated in Fig. 40 and the balloon 8 is caused to contract in the duodenal papilla PV as illustrated in Fig. 41.

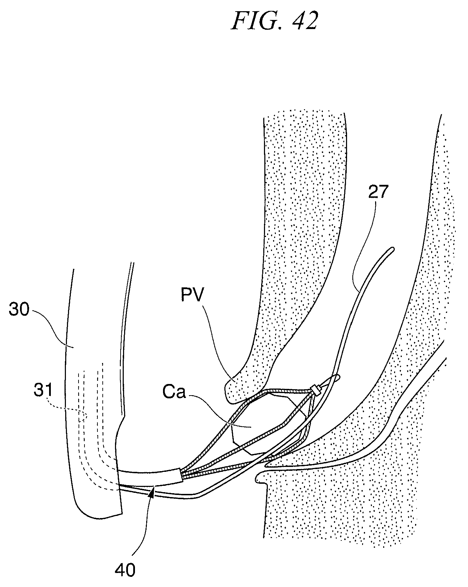

When the duodenal papilla PV expands, the catheter 1 is pulled out from the treatment tool channel 31 of the endoscope 30, a quarrying basket 40 or the like is attached to the endoscope 30 as illustrated in Fig. 42, and the basket 40 is introduced into the bile duct from the duodenal papilla PV to collect the calculus.

As described above, according to the catheter 1 of this embodiment, it is possible to easily perform a plurality of operations while holding the pressurizer 20.

Specifically, first, since the connecting portion 12 to which the three-way stopcock 24 is attached and the cylinder 21 of the pressurizer 20 are fixed by the fixing tool 16 to be relatively immovable, it is possible to perform attachment and detachment of the three-way stopcock 24 and switching of the cock of the three-way stopcock 24 using only the right hand while holding the pressurizer 20 in the left hand.

When a two-way stopcock is used instead of the three-way stopcock 24, the two-way stopcock and the pressurizer 20 are disconnected to discharge air to the outside of the pressurizer 20, instead of discharging the air in the pressurizer 20 to the outside from the open end of the three-way stopcock 24. At this time, it is also possible to perform detachment and attachment of the two-way stopcock and the pressurizer 20 using only the right hand.

Since the connector 13 serving as the fluid feed port and the cylinder 21 of the pressurizer 20 are fixed by the fixing tool 16 to be relatively immovable, it is possible to perform attachment and detachment of the syringe 26 for injecting a radiocontrast agent to and from the connector 13 using only the right hand while holding the pressurizer 20 in the left hand. Accordingly, even when the radiocontrast agent in the syringe 26 is used up during the treatment, it is possible to detach the syringe 26 and to easily replace the syringe with a preliminary syringe 26 filled with a radiocontrast agent.

When the radiocontrast agent is injected using the syringe 26, the operation section 10 of the catheter 1 and the cylinder 21 of the pressurizer 20 are fixed by the fixing tool 16 such that there is no relative movement between the pressurizer 20 and the syringe 26 and thus the force for pressing the syringe 26 can be received while the left hand holds the pressurizer 20. Accordingly, since the syringe 26 can be operated between the right hand and the left hand, it is possible to more easily perform fluid feed requiring force.

The operation section 10 and the cylinder 21 are fixed by the fixing tool 16 such that there is no relative movement between the proximal opening 14 serving as an entrance of the guide wire 27 and the cylinder 21 of the pressurizer 20. Accordingly, it is possible to perform insertion or pulling of the guide wire 27 into or from the proximal opening 14, delicate forward and backward movement, and a rotational operation using only the right hand without changing the left hand holding the pressurizer 20.

When it is necessary to replace the guide wire 27 with another type of guide wire 27 during the treatment, it is possible to easily perform the replacement using only the right hand.

Since the operation section 10 and the cylinder 21 of the pressurizer 20 are fixed such that there is no relative movement therebetween, it is possible to rapidly perform an operation of hooking a finger on the slider 17 to operate the knife wire 9.

In the direction in which the connection cord 29 is pushed into the plug 18, the operation section 10 and the cylinder 21 are fixed by the fixing tool 16 such that there is no relative movement between the body of the pressurizer 20 and the plug 18. Accordingly, when the connection cord 29 of the high-frequency power supply 28 is connected to the plug 18, it is possible to easily perform attachment and detachment of the connection cord 29 using only the right hand while holding the pressurizer 20 in the left hand.

Since the compliance of the balloon 8 (table indicating the relationship between the diameter of the balloon 8 and the pressure) is disposed at the position at which the scale of the gauge 25 of the pressurizer 20 is simultaneously visible, it is possible to easily check the first target pressure in expanding the balloon 8.

While an embodiment of the present invention has been described above in detail with reference to the accompanying drawings, the specific configurations thereof are not limited to the embodiment, and also include design modifications or the like without departing from the gist of the present invention.

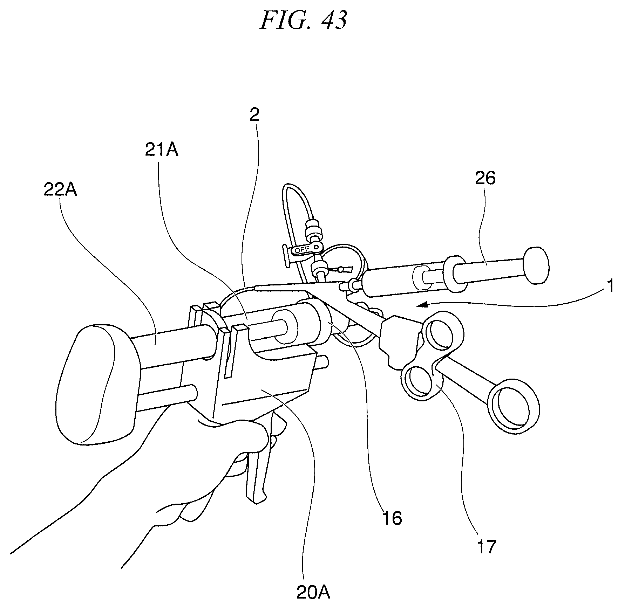

For example, in a hand gun type pressurizer 20A for linearly moving a plunger 22A relative to a cylinder 21A as illustrated in Fig. 43 instead of the pressurizer 20 described in the above-mentioned embodiment, the fixing tool 16 can also be attached to the cylinder 21 A.

### [Industrial Applicability]

In the above-mentioned embodiment, the proximal operation section of the multitask catheter having an expandable balloon can be attached to and detached from the body (a housing having a holding portion) of the pressurizer. Accordingly, since an assistant can hold the proximal operation section of the catheter while holding the pressurizer, the assistant can alternately perform the operation functions with his or her other hand.

### [Reference Signs List]

- 1: catheter
- 2: insertion section
- 3: inner sheath
- 4: first lumen
- 4a: first side hole
- 4b: second side hole
- 5: second lumen
- 6: third lumen
- 7: outer sheath
- 8: balloon
- 9: knife wire
- 10: operation section
- 11: body
- 12: connecting portion
- 13: connector
- 14: proximal opening
- 15: display portion
- 16: fixing tool
- 17: slider
- 18: plug
- 20: pressurizer
- 21: cylinder
- 22: plunger
- 23: connection tube
- 24: three-way stopcock
- 25: gauge
- 26: syringe
- 27: guide wire
- 28: high-frequency power supply
- 29: connection cord
- 30: endoscope
- 31: treatment tool channel

## Claims

1. A catheter comprising:
a tubular insertion section configured to be inserted into a body;
an expandable and contractible balloon that is disposed at a distal end portion of the insertion section to communicate with lumen of the insertion section;
a treatment section that is disposed at the distal end portion of the insertion section and configured to treat a biological tissue; and
an operation section that is disposed at a proximal end portion of the insertion section and configured to operate the balloon and the treatment section,
wherein the operation section includes
a connecting portion that is attached to a discharge port of a pressurizer capable of supplying a fluid to the balloon from the discharge port, and
a fixing tool which is configured to fix the operation section to the pressurizer to be detachable therefrom.

2. The catheter according to Claim 1, wherein the fixing tool has a C shape, and
the operation section includes a display portion in which at least three outer diameters of the balloon and internal pressures of the balloon corresponding to the at least three outer diameters are displayed on a substantial plane intersecting a plane perpendicular to a center line of a substantial cylinder formed by the C shape of the fixing tool at an angle of 45° or less to exhibit a one-to-one relationship between the outer diameter and the internal pressure.

3. The catheter according to Claim 2, wherein the relationship between the outer diameter of the balloon and the internal pressure of the balloon is nonlinear.

4. The catheter according to Claim 1, wherein the operation section includes a display portion in which at least three outer diameters of the balloon and internal pressures of the balloon corresponding to the at least three outer diameters are displayed to exhibit a one-to-one relationship between the outer diameter and the internal pressure,
the pressurizer includes a gauge indicating a pressure of the fluid to correspond to the pressure of the fluid, and
the fixing tool connects the operation section and the pressurizer to have a positional relationship in which display of the outer diameters and the internal pressures of the balloon in the display portion and the pressure displayed in the gauge are capable of simultaneously being visually recognized from a predetermined direction.

5. The catheter according to Claim 4, wherein the relationship between the outer diameter of the balloon and the internal pressure of the balloon is nonlinear.

6. The catheter according to any one of Claims 1 to 5, wherein the fixing tool is attachable to and detachable from the pressurizer and is selectively attachable to and detachable from any one of the pressurizer and an endoscope.

7. The catheter according to any one of Claims 1 to 6, wherein the treatment section includes a knife wire that is exposed to the outside of the insertion section at the distal end portion of the insertion section, that extends to the operation section via the lumen of the insertion section, and configured to incise a biological tissue by supplying a high-frequency current to the biological tissue,
the first lumen into which the knife wire is inserted is formed in the insertion section, and
the operation section includes
a slider that is fixed to a proximal end of the knife wire to cause the knife wire to move forward and backward in the axial direction of the knife wire, and
a plug that is fixed to the slider, is electrically connected to the knife wire, and is capable of being connected to a high-frequency power supply for supplying the high-frequency current to the knife wire.

8. The catheter according to any one of Claims 1 to 7, wherein the second lumen into which a guide wire is inserted is formed in the insertion section, and
the operation section includes a proximal opening which communicates with the second lumen at a proximal end portion of the second lumen and into which the guide wire is inserted.
